# EUROPEAN PATENT SPECIFICATION

(11) **EP 0 523 122 B1**
(45) Date of publication and mention of the grant of the patent: **17.04.1996**
(21) Application number: 91907101.9
(22) Date of filing: 28.03.1991
(51) Int. Cl.: C07C 69/30, C07C 69/33

(54) **ESTERS AND FLUIDS CONTAINING THEM**
ESTER UND DIESE ENTHALTENDE FLÜSSIGKEITEN
ESTERS ET LIQUIDES LES CONTENANT

(30) Priority: 30.03.1990 GB 9007193
(43) Date of publication of application: 20.01.1993
(73) Proprietor: EXXON CHEMICAL PATENTS INC., Florham Park New Jersey 07932 (US)
(72) Inventor: BENDA, Rainer, B-1150 Brussels (BE); GODWIN, Allen David, 1025 Singapore (SG); WALLAERT, Bernard Victor Raimond, F-76240 Bonsecours (FR)
(74) Representative: Bawden, Peter Charles
(86) International application number: GB9100491
(87) International publication number: WO9115455

(56) References cited:
- DE-A- 1 594 418
- DE-A- 2 302 918
- DE-A- 2 336 844
- FR-A- 2 254 633

## Description

The present invention relates synthetic esters and to lubricating compositions containing synthetic esters including aqueous lubricating compositions, the lubricants are especially useful as cutting fluids and hydraulic fluids, and the esters may be used as the oil phase of such fluids, or as additives thereto. The invention also relates to additive concentrates containing such esters for incorporation into such fluids.

Metal-working, or cutting fluids are employed as lubricants and coolants in operations such as, for example, cutting, drilling, milling, tapping, turning, reaming, and grinding. In such operations, it is customary to flood the tool and the workpiece with the fluid to carry off the heat generated. The fluids may be oils or aqueous based fluids containing an organic lubricant which are preferred since they make use of the superior heat transfer capacity of water as well as the lubricity of the organic component.

A problem encountered with aqueous based cutting fluids is there susceptibility to biodegradation. The various additives commonly present in such fluids, for example, corrosion inhibitors, antifoams, extreme pressure agents, antiwear agents, surfactants, and thickeners, generally act as nutrients for microorganisms and even if these organisms do not adversely affect the performance of a fluid, as by lowering its pH so that is become corrosive to metal or by breaking the emulsion, the proliferation of bacteria or fungi makes the fluid so foul smelling that it must be discarded. Regulations on effluent discharge have added to the desirability of reducing the susceptibility of cutting fluids to biodegradation.

There further remains a need to improve the lubricating properties of cutting fluids, for example by the addition of a specific oiliness agent in the case of solutions, and replacement of some of the proportion of mineral oil by a product having higher lubricating qualities in the case of emulsions. Still further, there remains a need for materials showing improved utility in microemulsions, now preferred because of their stability, the small size of the organic disperse phase particles reducing the tendency to coalesce.

We have now found that certain esters may be used to improve the properties, especially the lubricity, of such materials and that these esters have superior viscometric properties than comparable prior art esters such as those proposed in Japanese Patent Application 6372260 and United Kingdom Patent 1444826.

The present invention accordingly provides an ester of an aliphatic polyol having at least three hydroxy groups with a C₁₀ to C₁₅ branched aliphatic carboxylic acid containing on average more than one branch per molecule in which no more than 20% of the branching is on the 2 carbon atom.

The invention also provides concentrates for addition to oil to form an aqueous emulsion containing at least one such ester as part of the oil phase. The invention further provides a hydraulic fluid, and a metal-working fluid, especially an aqueous metal-working fluid, especially a cutting fluid, containing such ester or esters as part or all of the oil phase. The invention further provides the use of such an ester as a component, especially a lubricity enhancing component, in a cutting fluid.

The invention further provides the use of such esters as synthetic lubricants and as additives to provide lubricity to lubricating compositions which may be natural or synthetic.

The aliphatic polyol used in the production of the ester preferably contains from 4 to 10 more preferably 4 to 6 carbon atoms, there may be used, for example, one or more of trimethylol propane, pentaerythritol, glycerol, trimethylol ethane, sorbitol, dipentaerythritol and other oligomers of pentaerythritol. Of these polyols, those with at least three primary hydroxy groups such as pentaerythritol and trimethylol propane are preferred. It is also preferred that the polyol be fully esterified by the acid.

The aliphatic polyhydroxy compound may contain no functional groups other than hydroxy groups, or it may contain other functional groups, for example amine groups. It is preferably free, however, from sulphur-containing substituents.

The acids are preferably monocarboxylic acids of the formula 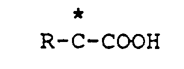 * being the 2 carbon atom and may be produced by the oxo process, in which one or more olefinically unsaturated hydrocarbons are hydroformylated by reaction with synthesis gas (hydrogen and carbon monoxide, usually in a weight ratio between 0.9 : 1 to 1.5 : 1) in the presence of a catalyst, generally a cobalt carbonyl, at elevated temperature and pressure (typically between 125°C and 175°C, and 150 to 300 atmospheres (150 to 300 bars)) to form an aldehyde with one more carbon atom than the feedstock olefin. The resulting aldehyde is subsequently oxidized to the corresponding acid. The feedstock olefin normally contains a substantial proportion of branched hydrocarbons but, in any event, the reactions taking place during the oxo process cause the product to have a highly branched structure and normally a mixture of such highly branched structures. For example, the tridecanoic acid is a mixture of trimethyldecanoic acids, tetramethylnonanoic acids and dimethyl undecanoic acids with branching distributed along the alkyl group.

Typically such acid mixtures contain less than 40% generally less than 20% and frequently less than 10% by weight of linear acids, less than 20% by weight of acids branched on the 2 carbon (the carbon atom α to the carboxy group) and the content of acids branched on carbon 3 or higher is equal to or higher than 40% by weight. The acids also preferably have, on average, more than 2, typically between 2 and 4 branches per molecule and the branches are generally short chain (C₁-C₄) usually methyl or ethyl groups. The branching is measured by proton NMR typically using a 220 megahertz machine.

Advantageously, the acid contains from 11 to 13 carbon atoms. For reasons of availability and economics, preferred acids are the C₁₁ and C₁₃ acids; the latter as normally available commercially, and as preferably used in the present invention, is a mixture of C₁₂ and C₁₃ acids.

The esters may be prepared by standard esterification techniques generally reacting one mole of acid for each hydroxy group in the aliphatic alcohol.

It is within the scope of the invention to use esters of acids that have been prepared by means other than the oxo process, provided they are multibranched acids within the definition of the invention, and more especially mixtures of multibranched acids, corresponding to those resulting from the oxo process. Such a procedure is not, however, at present economically favourable compared with the use of materials derived from the oxo process.

An emulsion concentrate within the invention comprises an aqueous dispersion of at least one ester according to the invention, and more especially comprises the ester, an emulsifier, and water, optionally together with other components normally present in cutting or hydraulic fluids. Such a concentrate will normally have from 10 to 80%, advantageously from 20 to 75%, preferably from 50 to 75%, by weight of active ingredients, the remainder being water. The concentrate may be readily diluted, as by the end user, with more water to give the hydraulic or cutting fluid of the invention, which latter may contain 0.1 to 15%, advantageously 0.5 to 10%, and preferably 0.5 to 5%, by weight of active ingredients, the remainder being water. The emulsions may be water in oil or oil in water emulsions, most preferred are the oil in water emulsions containing from 1 to 10 wt% preferably 2 to 5 wt% oil.

In general, the hydrophilic-lipophilic balance (HLB) of the emulsifier or emulsifiers is matched to that of the base oils or synthetic fluids present, which for a paraffinic oil is in the range of 9.0 and 10.5 while for a naphthenic oil it is between 10.5 and 11.5. Components of emulsifiers with HLB in the range of 5 to 15 may be employed to obtain an appropriate balance.

No change in formulation procedure is necessitated by the inclusion of an ester according to the invention; if any difficulty in dispersion of the ester is encountered a proportion or an increased proportion of emulsifier with HLB in the range of 9 to 11 may be employed.

As suitable emulsifiers there may be mentioned in particular nonionic and anionic surfactants. As examples, there may be mentioned condensates of an alkylene oxide with an organic compound containing an active hydrogen atom and, generally, at least 6 carbon atoms, for example, a condensate with an alcohol, a phenol, especially an alkyl phenol, a thiol, a primary or secondary amine, or a carboxylic or sulphonic acid or an amide thereof; block copolymers of ethylene and propylene oxide; ethoxylated alkly phosphates; alkyl sulphates and sulphonates; alkaryl sulphonates and salts thereof; derivatives of polyisobutylene succinic acids and anhydrides; fatty acid salts, and alkanolamines.

The components of the emulsion concentrate may be blended by the procedures normal in the art; no special technique is necessary. As is usual in the art, the components are blended together with stirring at ambient temperature, or slightly above, for example from 40°C to 60°C, for a time sufficient to give a uniform composition. The esters of the present invention being oil-soluble, the concentrate is preferably made by blending the ester with oil or hydrocarbon fluids, adding the emulsifier, and then the water phase containing the water-soluble additives. If necessary, the concentrate is subsequently blended with further water to give the desired active ingredient concentration.

The esters of the invention may be employed in cutting fluids of all types, aqueous systems include white emulsions and, especially, microemulsions, and also in oil-based or other hydrocarbon solvent based metal-working fluids, for example for use in rolling operations.

The esters may be employed as additives which impart mild extreme pressure and antiwear properties to the lubricant or they may be used as an oil replacement. The emulsion concentrate of the invention will therefore generally contain up to 75% advantageously from 2 to 70%, more preferably 5 to 70%, and most preferably from 10 to 65% by weight of the ester, based on the total weight of the components other than water, the proportion being at the higher end of the range where the ester replaces mineral oil to any extent. These concentrates are typically diluted into water to provide the final fluid containing from 3 to 15 wt% of the concentrate.

The emulsion concentrate, and the resulting hydraulic or metal-working fluid, may contain any or all of the usual additives, e.g., corrosion inhibitors, antifoams, extreme pressure agents, antiwear agents, pour point depressants, antioxidants, thickeners, and biocides. In the examples which follow, however, a simplified system, containing only oil, ester or comparison material, corrosion inhibitor, emulsifier and water, is employed.

The esters may also be used in water free systems, sometimes known as straight oils, there they may be used as an additive to provide lubricity where from 2 to 10% by weight enhances the lubricity of mineral oils or other synthetic oils such as polyalpha olefins. Alternatively the ester may be used to replace mineral oil in which case considerably larger quantities will be used.

### Example 1

### Preparation of the trisundecanoic acid ester of trimethylolpropane

To a 3 litre, 3 neck round bottom flask equipped with mechanical stirrer and Dean-Stark trap, are charged 1562.2 g (8.41 moles) of an undecanoic acid produced by oxidation of a C₁₁ aldehyde is produced by the oxo process (commercially available under the Trade Mark "Cekanoic Oil acid"), 3087.2 g (2.3 moles) trimethylol propane, and 12.2 g p-toluene sulphonic acid as a catalyst. The reaction mixture is purged with nitrogen, then heated to 100°C under a 300 mm Hg vacuum. Over a 3.5 hour period, the reaction temperature is increased to 140°C and the pressure reduced to 100 mm Hg. Water is collected in the Dean-Stark trap, a total of 114 ml being recovered. Heating is continued to collect unreacted acid, the mixture is cooled to 110°C, and 18.7 g sodium carbonate are added. The resulting mixture is stirred for 30 minutes, then 300 ml water are added with stirring and the mixture transferred to a separating funnel.

After the reaction mixture has been allowed to stand, the aqueous layer is drawn off, and then water washing is continued until the aqueous layer tests neutral. Remaining excess acid is removed by steam stripping at 140°C, 200 mm Hg. After stripping, the ester is filtered; yield 1255.8 g. Properties are given in Table 1, following Example 4, under the heading TMP C₁₁.

Proton NMR analysis of the aldehyde from which the acid was produced showed it to contain an average of 2.96 methyl containing branches, whilst on Carbon 13 NMR analysis showed that 10.9% branches on the 2 carbon atom, 49.9 on the 3, 23.6 on the 4 and 15.6 on the 5 or higher.

### Example 2

### Preparation of the tetraundecanoid acid ester of pentaerythritol

The same procedure was used as in Example 1, using 1499 g (8.06 moles) of the acid, 258.4 g (1.9 moles) of pentaerythritol, and 11.4 g of the catalyst. Properties are given in Table 1, under the heading PE C₁₁.

### Example 3

### Preparation of the tris tridecanoic ester of trimethylol propane

The same procedure was followed, using 1531.2 g (7.16 moles) of an oxo process tridecanoic acid obtained by oxidation of a C₁₃ aldehyde obtained by the oxo process, 301.5 g (2.25 moles) of trimethylol propane, and 11.9 g of the catalyst. Properties are given in Table 1, under the heading TMP C₁₃. Proton NMR analysis showed it to contain an average of 2.96 methyl containing branches whilst Carbon 13 analysis showed 8.4% of the branches to be in the 2 carbon atom, 47.7 on the 3 carbon, 26.9 on the 4 carbon and 17.0 on the 5 are higher.

### Exemple 4

### Preparation of the tetra tridecanoic acid ester of pentaerythritol

The ester was prepared using the same procedure as in Example 1, from tridecanoic acid and pentaerythritol. Properties are shown in Table 1, below, under the heading PE C₁₃.

**Table 1**

| | TMP C₁₁ | PE C₁₁ | TMP C₁₃ | PE C₁₃ |
|---|---|---|---|---|
| Acid Number mg KOH/g | 0.532 | 1.53 | 3.37 | 1.6 |
| | | | | |
| Colour FAC | < 1. | < 1. | < 1. | < 1. |
| | | | | |
| Saponification No., mg KOH/g | 261.9 | 271.7 | 239.8 | 248.8 |
| | | | | |
| Solidification Point, °C | < -38 | < -30 | < -36 | < -30 |
| | | | | |
| Viscosity, cSt 40°C | 83.62 | 160.2 | 111.16 | 218.9 |
| | | | | |
| Viscosity, cSt 100°C | 9.14 | 13.77 | 10.91 | 16.3 |

### Examples 5 to 7

Different concentrates were prepared according to compositions listed in Table 2. In this table, HVI 60 refers to a paraffinic oil sold by Shell and NYNAS T22 to a naphthenic oil sold by Nynas. The ester of Example 3 is referred to as TMP C₁₃; Synacto 3001 is an emulsifier commercially available from Exxon Chemical Limited being an oil solution of a mixture of salts of alkaryl sulphonic acids and salts of carboxylic acids of HLB approximately 10.8 and, in the formulations containing TMP C₁₃, Synacto 3002 is a similar emulsifier but with HLB approximately 9.2; Emulsogen BLM is a commercial fatty amide. The concentrates were diluted with 25 parts of water to make milky emulsions which were tested in the Falex lubrication tester, according to ASTM D 3233, the test being modified to measure friction coefficient and temperature rise at a load below breaking value, 400 kg, during a time interval of 5 minutes, following the procedure of IP 241 Method D.

**Table 2**

| (The proportions are given in parts by weight.) | | | | |
|---|---|---|---|---|
| Example No. | (comparative) | 5 | 6 | 7 |
| HVI 60 | -- | -- | 51.0 | 10.0 |
| NYNAS T22 | 81.0 | 71.0 | -- | -- |
| TMP C₁₃ | -- | 10.0 | 30.0 | 64.8 |
| Synacto 3001 | 16.0 | 15.0 | 12.8 | 9.0 |
| Synacto 3002 | -- | 1.0 | 3.2 | 13.5 |
| Emulsogen BLM | 3.0 | 3.0 | 3.0 | 2.7 |

| FALEX RESULTS | | | | |
|---|---|---|---|---|
| Friction Coefficient | 0.134 | 0.122 | 0.107 | 0.094 |
| Temperature Rise °C | 44 | 41 | 35 | 29 |

The results show the significant increase in lubrication achieved by replacing increasing proportions of base oil by the ester.

### Examples 8 to 11

To compare the esters according to the invention with known lubricity additives a standard formulation was prepared in the form of a microemulsion, the concentrate comprising, by weight precent, the components listed in Table 3. Synacto 2000 is a commercially available surfactant being an oil solution containing 50% by weight of salts of alkylaryl sulphonic acids with HLB of about 11, BDG is butyl diglycol. The corrosion inhibitor is an aqueous solution of alkanolamine salts of a mixture of tartaric and boric acids.

**Table 3**

| | |
|---|---|
| Nynas T22 | 21.4 |
| Synacto 2000 | 21.4 |
| BDG | 4.8 |
| Corrosion Inhibitor | 23.8 |
| Water | 23.8 |
| Ester or Comparison | 4.8 |

The concentrate was then mixed with water at a ratio of 1:25.

The materials used were as follows:
- Comparison A :: trimethylol propane (TMP) trioleate
- Example 8 :: TMP C₁₁
- Example 9 :: TMP C₁₃
- Example 10 :: PE C₁₁
- Example 11 :: PE C₁₃

A steel workpiece (steel designation 35 NCD 16) was ground with a grinding wheel (material designation 99 A60M6 V36) using the materials listed above as cutting fluid, and the ratio G of mass of material removed from the workpiece to that removed from the wheel was measured. The variation Rₐ in surface finish (Rₐ of the first piece, Rₐ of the 10th piece) in µm, from the workpiece, was also measured. The results are shown in Table 4.

**Table 4**

| | G | Rₐ |
|---|---|---|
| Comparison | 191 | 0.35 to 0.46 |
| Example 8 | 170 | 0.35 to 0.43 |
| Example 9 | 196 | 0.38 to 0.43 |
| Example 10 | 196 | 0.36 to 0.44 |
| Example 11 | 157 | 0.37 to 0.43 |

These results show an improvement in the consistency of the finish using the esters of the invention over that of the comparison.

### Examples 12 to 14

Using the same formulation as in Examples 8 to 11, the following Examples were used to test effectiveness of the esters according to the invention as components of cutting fluids in reaming of aluminium, under the following conditions: workpiece reference 5U 3Y 30; tool HSS.E at 5% cobalt; reaming of 750 holes; examination of the profile of every third hole by Perthometer M 3A; Rₐ is the average in µm of these results.

The results are show in Table 5.

**Table 5**

| | | Rₐ |
|---|---|---|
| Comparison A | TMP trioleate | 2.3 |
| Comparison B | Di(tridecyl) phthalate | 3.2 |
| Example 12 | TMP C₁₁ | 2.8 |
| Example 13 | TMP C₁₃ | 2.35 |
| Example 14 | PE C₁₁ | 2.6 |

These results show that the esters of the invention give significantly better performance than a diester in surface finish.

### Examples 15 to 17

The procedure of Examples 5 to 7 was followed, to make emulsion concentrates as shown in Table 6. These were then diluted with 25 parts of water to give a micro-emulsion. Table 6 also shows the Falex results.

**Table 6**

| Example No. | (comparative) | 15 | 16 | 17 |
|---|---|---|---|---|
| NYNAS T22 | 25.00 | 23.75 | 22.00 | -- |
| Corrosion Inhibitor | 20.00 | 19.00 | 18.00 | 20.00 |
| Synacto 3001 | 25.00 | 23.75 | 16.10 | 10.40 |
| Synacto 3002 | -- | -- | 6.40 | 14.60 |
| Water | 30.00 | 28.50 | 27.00 | 25.00 |
| TMP C₁₃ | -- | 5.00 | 10.00 | 30.00 |

| FALEX RESULTS | | | | |
|---|---|---|---|---|
| Friction Coefficient | 0.115 | 0.105 | 0.100 | 0.092 |
| Temperature Rise °C | 37 | 33 | 29 | 27 |

The results show that, using the ester of the invention, there may be formulated a highly effective synthetic emulsion containing essentially no mineral oil (less than 1% mineral oil being brought into the concentrate from Synacto 3001 and 3002).

## Claims

1. An ester composition of a C₁₀ to C₁₅ branched aliphatic carboxylic acid containing on average more than one branch per molecule in which no more than 20% of the branching is on the 2 carbon atom, and an aliphatic alcohol having at least three hydroxy groups.

2. An ester composition as claimed in claim 1, wherein the acid has been formed by the oxo process.

3. An ester composition as claimed in claim 1 or claim 2, derived from a C₁₁ acid.

4. An ester composition as claimed in claim 1 or claim 2, derived from a C₁₃ acid.

5. An ester composition as claimed in any of claims 1 to 4 in which the acid contains between 2 and 4 branches.

6. An ester composition as claimed in any of claims 1 to 5 in which the acid is a mixture of acids containing less than 40% by weight linear acid, and the content of acids branched on the 3 carbon and higher is equal to or higher than 40%.

7. An ester composition as claimed in any of claims 1 to 6 in which the aliphatic alcohol contains at least three primary hydroxy groups.

8. An ester composition as claimed in any one of claims 1 to 7 derived from trimethylol propane.

9. An ester composition as claimed in any one of claims 1 to 7 derived from pentaerythritol.

10. Trimethylol propane triundecanoate, the undecanoic acid being derived from a C₁₁ oxo process aldehyde.

11. Trimethylol propane tri-tridecanoate, the tridecanoic acid being derived from a C₁₃ oxo process aldehyde.

12. Pentaerythritol tetraundecanoate, the undecanoic acid being derived from a C₁₁ oxo process aldehyde.

13. Pentaerythritol tetra-tridecanoate, the tridecanoic acid being derived from a C₁₃ oxo process aldehyde.

14. A hydraulic fluid or lubricant comprising an ester composition as defined in any one of claims 1 to 13.

15. A cutting fluid comprising water, and emulsifying agent, and an ester composition as defined in any one of claims 1 to 13.

16. A cutting fluid as claimed in claim 15, which also comprises a mineral or synthetic oil.

17. A cutting fluid as claimed in claim 15, substantially free from mineral oil.

18. An emulsion concentrate comprising an ester composition as defined in any one of claims 1 to 13, an emulsifying agent, and water.

19. An emulsion concentrate as claimed in claim 18, wherein the ester composition represents up to 75% of the total weight of the components other than water.

20. An emulsion concentrate as claimed in claim 18, wherein the ester composition represents 2 to 70% of the total weight of the components other than water.

21. An emulsion concentrate as claimed in claim 18 wherein the ester composition represents 10 to 65% of the total weight of components other than water .

22. A method of metal working which uses as a lubricant or cutting fluid a composition as claimed in any one of claims 14 to 21.

23. The use of an ester composition as defined in any one of claims 1 to 13 as a lubricant aid in a cutting fluid.

## Patentansprüche

1. Esterzusammensetzung einer verzweigten aliphatischen C₁₀- bis C₁₅-Carbonsäure, die durchschnittlich mehr als eine Verzweigung pro Molekül enthält, wobei sich nicht mehr als 20 % der Verzweigung an dem 2-Kohlenstoffatom befinden, und eines aliphatischen Alkohols mit mindestens drei Hydroxygruppen.

2. Esterzusammensetzung nach Anspruch 1, bei der die Säure nach dem Oxoverfahren hergestellt worden ist.

3. Esterzusammensetzung nach Anspruch 1 oder Anspruch 2, die von einer C₁₁-Säure abgeleitet ist.

4. Esterzusammensetzung nach Anspruch 1 oder Anspruch 2, die von einer C₁₃-Säure abgeleitet ist.

5. Esterzusammensetzung nach einem der Ansprüche 1 bis 4, bei der die Säure zwischen 2 und 4 Verzweigungen enthält.

6. Esterzusammensetzung nach einem der Ansprüche 1 bis 5, bei der die Säure eine Mischung von Säuren ist, die weniger als 40 Gew.% lineare Säure enthält, und der Gehalt an am 3-Kohlenstoffatom und höheren Kohlenstoffatomen verzweigten Säuren gleich oder höher als 40 % ist.

7. Esterzusammensetzung nach einem der Ansprüche 1 bis 6, bei der der aliphatische Alkohol mindestens drei primäre Hydroxygruppen enthält.

8. Esterzusammensetzung nach einem der Ansprüche 1 bis 7, die von Trimethylolpropan abgeleitet ist.

9. Esterzusammensetzung nach einem der Ansprüche 1 bis 7, die von Pentaerythrit abgeleitet ist.

10. Trimethylolpropan-tri-undecanoat, bei dem die Undekansäure aus einem C₁₁-Aldehyd aus dem Oxoverfahren stammt.

11. Trimethylolpropan-tri-tridecanoat, bei dem die Tridecansäure aus einem C₁₃-Aldehyd aus dem Oxoverfahren stammt.

12. Pentaerythrit-tetra-undecanoat, bei dem die Undekansäure aus einem C₁₁-Aldehyd aus dem Oxoverfahren stammt.

13. Pentaerythrit-tetra-tridecanoat, bei den die Tridecansäure aus einem C₁₃-Aldehyd aus dem Oxoverfahren stammt.

14. Hydraulikflüssigkeit oder Schmierstoff, die bzw. der eine Esterzusammensetzung gemäß einem der Ansprüche 1 bis 13 umfaßt.

15. Schneidflüssigkeit, die Wasser und Emulgator und eine Esterzusammensetzung gemäß einem der Ansprüche 1 bis 13 umfaßt.

16. Schneidflüssigkeit nach Anspruch 15, die auch ein Mineralöl oder synthetisches öl umfaßt.

17. Schneidflüssigkeit nach Anspruch 15, die im wesentlichen frei von Mineralöl ist.

18. Emulsionskonzentrat, das eine Esterzusammensetzung gemäß einem der Ansprüche 1 bis 13, einen Emulgator und Wasser umfaßt.

19. Emulsionskonzentrat nach Anspruch 18, bei dem die Esterzusammensetzung bis zu 75 % des Gesamtgewichts der von Wasser verschiedenen Komponenten ausmacht.

20. Emulsionskonzentrat nach Anspruch 18, bei dem die Esterzusammensetzung 2 bis 70 % des Gesamtgewichts der von Wasser verschiedenen Komponenten ausmacht.

21. Emulsionskonzentrat nach Anspruch 18, bei dem die Esterzusammensetzung 10 bis 65 % des Gesamtgewichts der von Wasser verschiedenen Komponenten ausmacht.

22. Verfahren zur Metallbearbeitung, das als Schmierstoff oder Schneidflüssigkeit eine Zusammensetzung gemäß einem der Ansprüche 14 bis 21 verwendet.

23. Verwendung einer Esterzusammensetzung gemäß einem der Ansprüche 1 bis 13 als Schmierhilfsmittel in einer Schneidflüssigkeit.

## Revendications

1. Composition d'ester d'un acide carboxylique aliphatique ramifié en C₁₀ à C₁₅ contenant en moyenne plus d'une ramification par molécule, dans laquelle pas plus de 20 % des ramifications sont présentes sur le deuxième atome de carbone, et d'un alcool aliphatique ayant au moins trois groupes hydroxy.

2. Composition d'ester suivant la revendication 1, dans laquelle l'acide a été formé par le procédé oxo.

3. Composition d'ester suivant la revendication 1 ou la revendication 2, dérivée d'un acide en C₁₁.

4. Composition d'ester suivant la revendication 1 ou la revendication 2, dérivée d'un acide en C₁₃.

5. Composition d'ester suivant l'une quelconque des revendications 1 à 4, dans laquelle l'acide contient 2 à 4 ramifications.

6. Composition d'ester suivant l'une quelconque des revendications 1 à 5, dans laquelle l'acide consiste en un mélange d'acides contenant moins de 40 % en poids d'acide linéaire, et la teneur en acides ramifiés sur le troisième atome de carbone et les atomes suivants est égale ou supérieure à 40 %.

7. Composition d'ester suivant l'une quelconque des revendications 1 à 6, dans laquelle l'alcool aliphatique contient au moins trois groupes hydroxy primaires.

8. Composition d'ester suivant l'une quelconque des revendications 1 à 7, dérivée du triméthylolpropane.

9. Composition d'ester suivant l'une quelconque des revendications 1 à 7, dérivée du pentaérythritol.

10. Triundécanoate de triméthylolpropane, dont l'acide undécanoïque est dérivé d'un aldéhyde en C₁₁ de procédé oxo.

11. Tri-tridécanoate de triméthylolpropane, dont l'acide tridécanoïque est dérivé d'un aldéhyde en C₁₃ de procédé oxo.

12. Tétraundécanoate de pentaérythritol, dont l'acide undécanoïque est dérivé d'un aldéhyde en C₁₁ de procédé oxo.

13. Tétra-tridécanoate de pentaérythritol, dont l'acide tridécanoïque est dérivé d'un aldéhyde en C₁₃ de procédé oxo.

14. Fluide hydraulique ou lubrifiant comprenant une composition d'ester répondant à la définition suivant l'une quelconque des revendications 1 à 13.

15. Fluide de coupe comprenant de l'eau, un agent émulsionnant et une composition d'ester répondant à la définition suivant l'une quelconque des revendications 1 à 13.

16. Fluide de coupe suivant la revendication 15, qui comprend également une huile minérale ou une huile synthétique.

17. Fluide de coupe suivant la revendication 15, pratiquement dépourvu d'huile minérale.

18. Concentré d'émulsion comprenant une composition d'ester répondant à la définition suivant l'une quelconque des revendications 1 à 13, un agent émulsionnant et de l'eau.

19. Concentré d'émulsion suivant la revendication 18, dans lequel la composition d'ester représente jusqu'à 75 % du poids total des constituants autres que l'eau.

20. Concentré d'émulsion suivant la revendication 18, dans lequel la composition d'ester représente 2 à 70 % du poids total des constituants autres que l'eau.

21. Concentré d'émulsion suivant la revendication 18, dans lequel la composition d'ester représente 10 à 65 % du poids total des constituants autres que l'eau.

22. Procédé d'usinage de métaux, qui utilise comme lubrifiant ou fluide de coupe une composition suivant l'une quelconque des revendications 14 à 21.

23. Utilisation d'une composition d'ester répondant à la définition suivant l'une quelconque des revendications 1 à 13 comme adjuvant de lubrification dans un fluide de coupe.
